# EUROPEAN PATENT APPLICATION

(11) **EP 1 635 277 A2**
(43) Date of publication of application: **15.03.2006**
(21) Application number: 05016347.6
(22) Date of filing: 27.07.2005
(51) Int. Cl.: G06F 19/00

(54) **System and methods for visualizing and manipulating multiple data values with graphical views of biological relationships**

(30) Priority: 27.08.2004 US 928494
(71) Applicant: Agilent Technologies, Inc. (a Delaware corporation), Palo Alto, California 94306-2024 (US)
(72) Inventor: Kuchinsky, Allan, San Francisco CA 94025 (US); Kincald, Robert, Half Moon Bay CA 94019 (US)
(74) Representative: Schoppe, Fritz

(57) **Abstract**

Methods, systems and computer readable media for visualizing multiple data values adjacent graphical representations of entities (402) in a diagram (100,200,400) representing biological relationships between the entities. A diagram of interconnected entities representing biological relationships between the entities is displayed. A data set having rows of data values, each row containing values representing a single entity is provided, wherein at least some of the entities are represented on the diagram. At least one row of data values from the dataset is overlaid (404,414) on the displayed diagram (100,200,400) such that the row of data values appears adjacent the entity (402) on the diagram that matches the entity in the data set that the row of data characterizes. The display (404,414) of the row of data values is scaled so that components of the display are dimensionally proportional to numerical values of the data values taken from the data set.

## Description

### CROSS-REFERENCE

This application is a continuation-in-part application of Application Serial No. 10/155,616, filed May 22, 2002, pending, which is incorporated herein, in its entirety, by reference thereto, and to which application we claim priority under 35 USC §120. This application is also a continuation-in-part application of Application Serial No. 10/403,762, filed March 31, 2003, pending, which claims the benefit of U.S. Provisional Application No. 60/402,566, filed August 8, 2002, now abandoned. Application Serial Nos. 10/403,762 and 60/402,566 are incorporated herein, in their entireties, by reference thereto, and to which applications we claim priority under 35 USC §120 and 35 USC §119, respectively.

### FIELD OF THE INVENTION

The present invention pertains to software systems supporting the activities of organizing, using, and sharing diverse biological information.

### BACKGROUND OF THE INVENTION

The advent of new experimental technologies that support molecular biology research have resulted in an explosion of data and a rapidly increasing diversity of biological measurement data types. Examples of such biological measurement types include gene expression from DNA microarray or Taqman experiments, CGH data, aCGH data, protein identification from mass spectrometry or gel electrophoresis, cell localization information from flow cytometry, phenotype information from clinical data or knockout experiments, genotype information from association studies and DNA microarray experiments, etc. This data is rapidly changing. New technologies frequently generate new types of data.

Biologists use this experimental data and other sources of information to piece together interpretations and form hypotheses about biological processes. Such interpretations and hypotheses can be represented by narrative descriptions or visual abstractions such as pathway diagrams. To build interpretations and hypotheses, biologists need to view these diverse data from multiple perspectives. In particular, it is very important to validate the possible interpretations and hypotheses against the detailed, experimental results, in order to test whether the interpretations/hypotheses are supported by the actual data. An example of this would be to validate, test, or illustrate a putative pathway, represented in a pathway diagram, against gene expression data.

Although some tools have been developed for overlaying a specific type of data onto a viewer, they are very limited in their approach and do not facilitate the incorporation of diverse data types whatsoever. For example, a tool called EcoCyc [http://ecocyc.org]. is capable of overlaying gene expression data on pathways, but is limited to only gene expression data. Another example known as GeneSpring, by Silicon Genetics [http://www.sigenetics.com], is available for overlaying gene expression data on genomic maps, but again, is limited to this specific application. GeneSpring further has an option to "color by all s conditio" on a pathway. In a case described on the Silicon Genetics website http://www.silicongenetics.com/cgi/SiG.cgi/Products/GeneSpring/index.smf, the "pathway" is actually a cell cycle diagram, and the experiments (conditions) are shown simultaneously as a continuous heatmap representing the values for the included experiments. However, using color alone is not optimal for accurate numerical comparisons. See also http://www.silicongenetics.com/cgi/SiG.cgi/Support/GeneSpring/GSnotes/pathw ays.smf and http://www.silicongenetics.com/cgi/TNgen.cgi/GeneSpring/GSnotes/Notes/what path Better techniques are needed to graphically represent the magnitudes of the underlying data values represented in a visualization.

Vector Pathblazer, by Invitrogen Life Technologies offers software to find pathways and reactions related to differentially expressed genes, see http://www.invitrogen.com/content.cfm?pageid=10360. Gene ontology annotations may be imported from the public domain, and connections between two pathways, or a pathway and a given component may be searched for. Important pathways may be shown with expression levels although there does not appear to be the ability to overlay gene expression data over the genes displayed in a pathway, see http://www.invitrogen.com/content.cfm?pageid=10363 and http://www.invitrogen.com/imgLibrary/sendExpData2 crop.gif:

Because of the vast scale and variety of sources and formats of these various types of data, an enormous number of variables must be compared and tested to formulate and validate hypotheses. Thus, there is a need for new and better tools that facilitate the comparisons of experimental data in conjunction with pathway representations for formulating and validating/invalidating hypotheses. Further, there is a particular need for tools to compare differential data values across multiple conditions, in the context of a biological process or molecular function.

### SUMMARY OF THE INVENTION

Methods, systems and computer readable media are provided for visualizing multiple data values adjacent to graphical representations of entities in a diagram representing biological relationships between the entities. A diagram of interconnected entities representing biological relationships between the entities is displayed. A data set having rows of data values, each row containing values representing a single entity is provided for access by the system. At least one display of a row of data values from the dataset is overlaid on the displayed diagram such that the row of data values appears adjacent the entity on the diagram that matches the entity in the data set that the row of data characterizes. The display of the row of data values is scaled so that components of the display are dimensionally proportional to numerical values of the data values taken from the data set.

A visualization graphic is disclosed for representing a row of data values from a dataset ori a displayed diagram such that the row of data values appears adjacent an entity on the diagram that matches the entity in the data set that the row of data characterizes. The visualization graphic comprises a graphical representation of each data value in the row of data values represented, wherein each graphical representation is scaled dimensionally proportional to a numerical value of the data value that it represents, as taken from the data set.

The present invention also covers forwarding, transmitting and/or receiving results from any of the methods described herein.

These and other advantages and features of the invention will become apparent to those persons skilled in the art upon reading the details of the methods, systems and computer readable media as more fully described below.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

Fig. 1 shows an example of color encoding data values to provide a "heat map" view wherein experimental data values are encoded on a color scale.

Fig. 2 shows a view of gene expression data from a single experimental condition having been overlaid on an interactive network diagram.

Fig. 3 shows the same network diagram as in Fig. 2, but with data from a different experimental condition overlaid thereon.

Fig. 4 shows one implementation of the present invention in which multiple data values (e.g., experimental data values) from multiple experimental conditions are overlaid on nodes of a network diagram.

Fig. 5 shows a magnified view of a node from Fig. 4 and its associated heat strip overlay.

Fig. 6 is a magnified view of a node from Fig, 4 which is the same as the node shown in Fig. 5, but where the associated overlay is represented in an alternative "line graph" style representation.

Fig. 7 shows representations of interlinked views according to the present invention, and cursors used to manipulate and navigate in the views.

Fig. 8 illustrates a typical computer system that may be used in processing events described herein.

### DETAILED DESCRIPTION OF THE INVENTION

Before the present systems, methods and computer readable media are described, it is to be understood that this invention is not limited to particular examples described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value; to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a pathway" includes a plurality of such pathways and reference to "the gene" includes reference to one or more genes and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DEFINITIONS

The term "cell", when used in the context describing a data table or heat map, refers to the data value at the intersection of a row and column in a spreadsheet-like data structure or heat map; typically a property/value pair for an entity in the spreadsheet, e.g. the expression level for a gene.

"Color coding" refers to a software technique which maps a numerical or categorical value to a color value, for example representing high levels of gene expression as a reddish color and low levels of gene expression as greenish colors, with varying shade/intensities of these colors representing varying degrees of expression. Color-coding is not limited in application to expression levels, but can be used to differentiate any data that can be quantified, so as to distinguish relatively high quantity values from relatively low quantity values. Additionally, a third color can be employed for relatively neutral or median values, and shading can be employed to provide a more continuous spectrum of the color indicators.

The term "down-regulation" is used in the context of gene expression, and refers to a decrease in the amount of messenger RNA (mRNA) formed by expression of a gene, with respect to a control.

The term "gene" refers to a unit of hereditary information, which is a portion of DNA containing information required to determine a protein's amino acid sequence.

"Gene expression" refers to the level to which a gene is transcribed to form messenger RNA molecules, prior to protein synthesis.

"Gene expression ratio" is a relative measurement of gene expression, wherein the expression level of a test sample is compared to the expression level of a reference sample.

A "gene product" is a biological entity that can be formed from a gene, e.g. a messenger RNA or a protein.

A "heat map" or "heat map visualization" is a visual representation of a tabular data structure of gene expression values, wherein color-codings are used for displaying numerical values. The numerical value for each cell in the data table is encoded into a color for the cell. Color encodings run on a continuum from one color through another, e.g. green to red or yellow to blue for gene expression values. The resultant color matrix of all rows and columns in the data set forms the color map, often referred to as a "heat map" by way of analogy to modeling of thermodynamic data.

A "heat strip" or "heat strip visualization" is a visual representation of a row of data structure from a tabular data structure such as a heat map. Typically, the heat strip visualization displays gene expression values from a single gene, but it is not limited to representation of gene expression values, as other data values may be similarly represented. Color-codings are used for displaying numerical values in the same way as described with regard to heat maps. Additionally, vertical bars of the heat strip have lengths that vary in proportion to the data values that the bars represent.

A "hypothesis" refers to a provisional theory or assumption set forth to explain some class of phenomenon.

An "item" refers to a data structure that represents a biological entity or other entity. An item is the basic "atomic" unit of information in the software system.

A "microarray" or "DNA microarray" is a high-throughput hybridization technology that allows biologists to probe the activities of thousands of genes under diverse experimental conditions. Microarrays function by selective binding (hybridization) of probe DNA sequences on a microarray chip to fluorescently-tagged messenger RNA fragments from a biological sample. The amount of fluorescence detected at a probe position can be an indicator of the relative expression of the gene bound by that probe.

The term "normalize" refers to a technique employed in designing database schemas. When designing efficiently stored relational data, the designer attempts to reduce redundant entries by "normalizing" the data, which may include creating tables containing single instances of data whenever possible. Fields within these tables point to entries in other tables to establish one to one, one to many or many to many relationships between the data. In contrast, the term "de-normalize" refers to the opposite of normalization as used in designing database schemas. De-normalizing means to flatten out the space efficient relational structure resultant from normalization, often for the purposes of high speed access that avoid having to follow the relationship links between tables. In another context, "normalization" refers to the transformation of data values to accommodate for a wide dynamic range in values across a dataset. In this usage, different data values can be compared against a compatible scale. For example, a "row normalized" display of heat map values represents each value in the row as a ratio of the value against the mean or median of the values in the row. This type of normalization can accommodate vastly different levels of expression that may occur in a data set.

The term "promote" refers to an increase of the effects of a biological agent or a biological process.

A "protein" is a large polymer having one or more sequences of amino acid subunits joined by peptide bonds.

The term "protein abundance" refers to a measure of the amount of protein in a sample; often done as a relative abundance measure vs. a reference sample.

"Protein/DNA interaction" refers to a biological process wherein a protein regulates the expression of a gene, commonly by binding to promoter or inhibitor regions.

"Protein/Protein interaction" refers to a biological process whereby two or more proteins bind together and form complexes.

A "sequence" refers to an ordered set of amino acids forming the backbone of a protein or of the nucleic acids forming the backbone of a gene.

The term "overlay" or "data overlay" refers to a user interface technique for superimposing data from one view upon data in a different view; for example, overlaying gene expression ratios on top of a compressed matrix view, or overlaying a heat strip visualization on a pathway visualization, such that the heat strip visualization is displayed adjacent a node the represent the entity that the data in the heat strip visualization is characterizing.

A "spreadsheet" is an outsize ledger sheet simulated electronically by a computer software application; used frequently to represent tabular data structures.

The term "up-regulation", when used to describe gene expression, refers to an increase in the amount of messenger RNA (mRNA) formed by expression of a gene, with respect to a control.

The term "UniGene" refers to an experimental database system which automatically partitions DNA sequences into a non-redundant sets of gene-oriented clusters. Each UniGene cluster contains sequences that represent a unique gene, as well as related information such as the tissue types in which the gene has been expressed and chromosome location.

The term "view" refers to a graphical presentation of a single visual perspective on a data set.

The term "visualization" or "information visualization" refers to an approach to exploratory data analysis that employs a variety of techniques which utilize human perception; techniques which may include graphical presentation of large amounts of data and facilities for interactively manipulating and exploring the data.

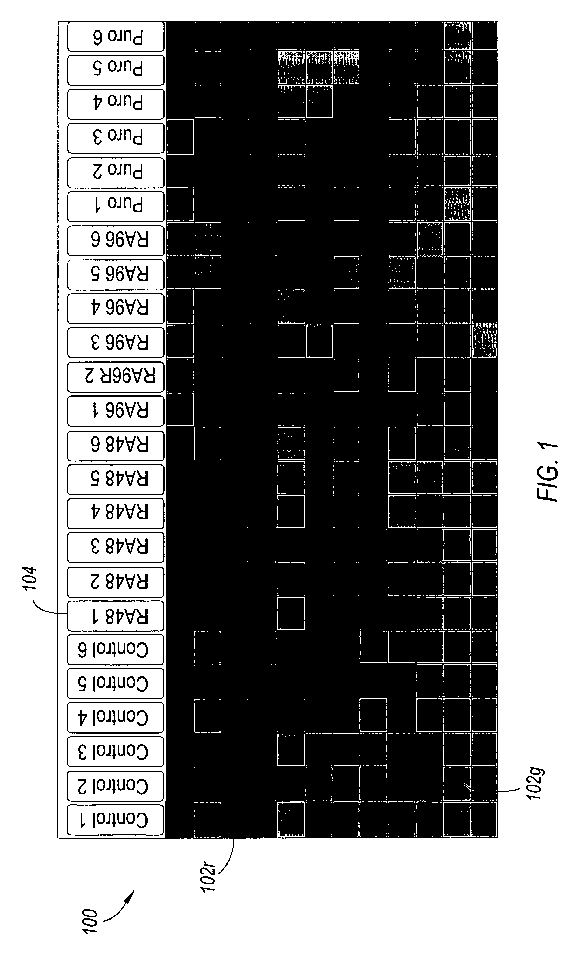

Fig. 1 shows an example of color encoding data values to provide a "heat map" view 100 wherein experimental data values are encoded on a color scale. In this example, the experimental values that are color coded are related to gene expression, and the color encodings rang from green 102g (representing a down-regulated gene) to red 102r (representing an up-regulated gene). The intensity and hue of the coloring is also scaled to the degree of up-regulation or down-regulation, such that a relatively more up-regulated value is brighter red and a relatively less up-regulated value is darker red. Neutral genes are color coded black, and the green and red color scales blend to black as the down- regulation values and up-regulation values approach neutral, respectively. As shown, one row of color coded cells represents gene expression values for one gene over a multiplicity of experimental conditions, each experimental condition being labeled by a column header 104. Thus, each row contains values for a single gene across a plurality of experiments, and each column contains values for a plurality of genes relative to the same experiment. Co-pending, commonly owned application Serial No. 10/403,762 discloses in detail the display and manipulation of experimental data values in heat map style representations such as shown in the example of Fig. 1.

Co-pending, commonly owned application Serial No.10/155,616 discloses generalized methods and systems for visualizing correlations of data and hypotheses through a mechanism called generalized data overlays. In a data overlay, data from one view is encoded (e.g., color coded) and superimposed upon data items in a different view.

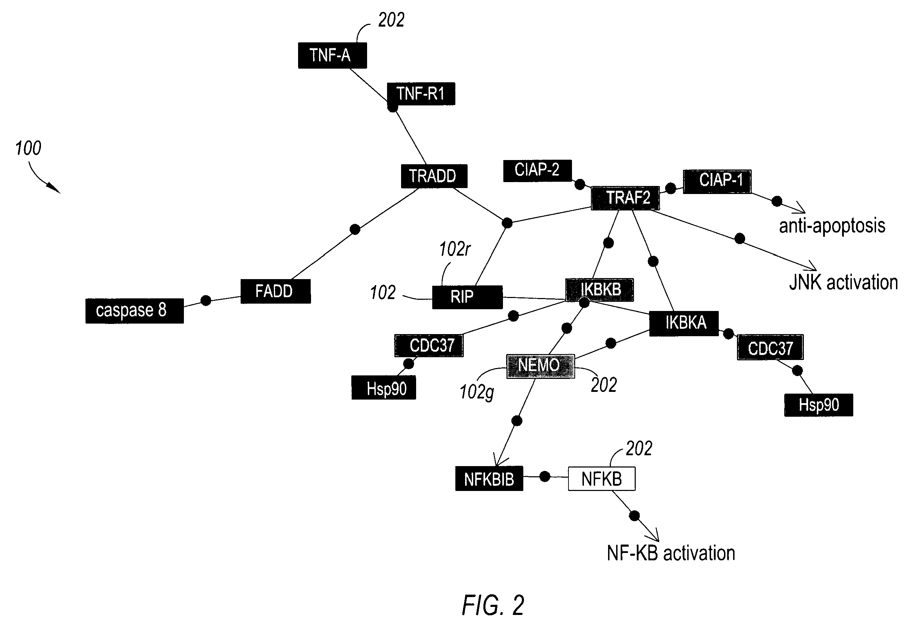

Fig. 2 shows a view of gene expression data having been overlaid on an interactive network diagram 200 of the type described in more detail in co-pending application Serial No. 10/155,616. The gene expression values that are overlaid on the graphical representations 202 for genes in the diagram 200 are color-encoded or color coded in similar fashion to that described above with regard to the heat map of Fig. 1. Thus, for example, gene "NEMO" 202 is color coded green 102g, indicating that this gene is down-regulated for the experiment that is currently being displayed on diagram 200, and gene "RIP" 200 is color coded red 102r, indicating that this gene is up-regulated for the experiment that is currently being displayed on diagram 200. When a gene is not color-coded, or is "blank" or white, such as "NFKB" 202 in Fig. 2, this indicates that there was no experimental value provided for that gene with respect to the experiment that is currently overlaid. Like Fig. 1, the intensity and hue of the coloring of the color coded overlays is also scaled to the degree of up-regulation or down-regulation, such that a relatively more up-regulated value is brighter red and a relatively less up-regulated value is darker red, and a relatively more down-regulated value is brighter green compared to a relatively less down-regulated value that is darker green. Neutral genes are color coded black, and the green and red color scales blend to black as the down- regulation values and up-regulation values approach neutral, respectively.

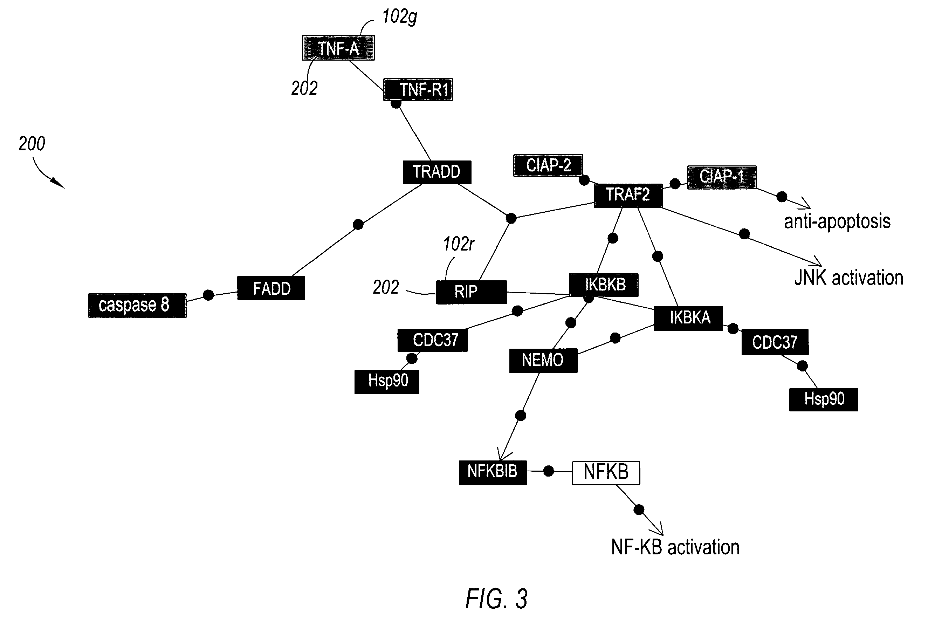

Fig. 3 shows the same network diagram 200 as in Fig. 2, but with a different experimental condition overlaid thereon. When comparing the two views, it can be readily observed, for example, that the value for "TNF-A" 202 in Fig. 3 is more down-regulated for that in Fig. 2, since the color coding for this gene is significantly brighter green than for that in Fig. 2. Similarly, it can be observed that the value for "RIP" 202 in Fig. 3 is significantly less up-regulated than for that in Fig. 2, since the color coding for this gene in Fig. 3 is darker red than for that in Fig. 2.

Visualizations of the types described with regard to Figs. 2 and 3 above are useful adjuncts to the heat map style visualization of Fig. 1, in that thy can display an experimental data value in it biological context, by showing where this value is occurring within a functional pathway. However, these types of visualizations do not provide a good sense of the variability of data values over experimental conditions, since overlays must be viewed as one experiment at a time, which makes it difficult to compare across experiments. Additionally, it is difficult to compare subtle differences in experimental values, e.g., difficult to interpret a difference in data values for one gene that shows two sh ades of red for two different experimental conditions, wherein the shades of red are not too far different from one another.

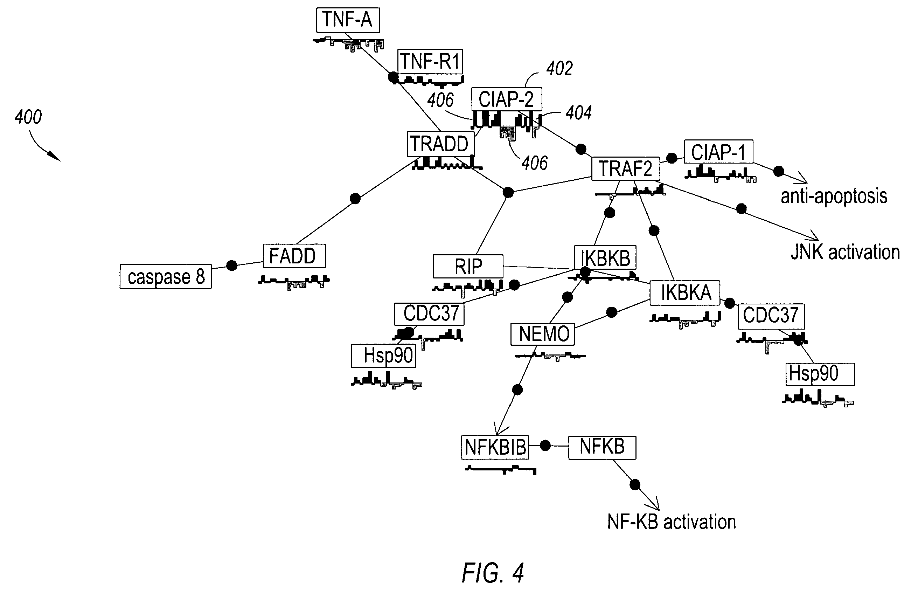

Fig. 4 shows one implementation of the present invention in which multiple data values (e.g., experimental data values) are overlaid on nodes of a network diagram. In this example, the same pathway diagram was used as in the visualizations described above with regard to Figs. 2 and 3. In view 400 however, the "nodes" or graphical representations 402 of the genes are not color coded, in contrast with what is shown in Figs. 2-3. Rather, a heat strip 404 is overlaid adjacent node 402 to represent data values from multiple experimental values for that gene, i.e., a value for each of a plurality of experiments regarding the gene represented by that particular node 402. Additionally, the dimensions (e.g., height, width, coordinate position) of the overlay elements (such as heat strips, in this example) may be used to represent difference in values, so that a user can more easily visually identify such differences when viewing such a visualization.

For example, heat strip 402 can be thought of or described as representing the superimposition of one row of a heat map representation (such as heat map representation 100 for example) underneath a node (such as node 402, for example) in a network diagram (such as diagram 400, for example), wherein the node represents the equivalent biological entity that is represented by the row of the heat map. In the heat strip 404 visualization, the rectangular area beneath the node 402 of the visualization where heat strip 404 is to be overlaid is divided into a set of vertical strips of equal width. Each strip will contain a color coded vertical bar representative of one cell in the row from the heat map, respectively. The width of each bar is equal to the width of the rectangular display area, in pixels, divided by the number of columns in the corresponding heat map. The vertical bars extend either upwardly, downwardly, or not at all from an imaginary centerline that bisects the rectangular area horizontally. Up-regulated values are encoded as red bars that extend upwardly from the centerline and down-regulated values are encoded as green bars that extend downwardly from the centerline. Neutral values are represented as a black horizontal line having the same width as the vertical bars, but no height, so that the neutral values do not extend upwardly or downwardly from the centerline.

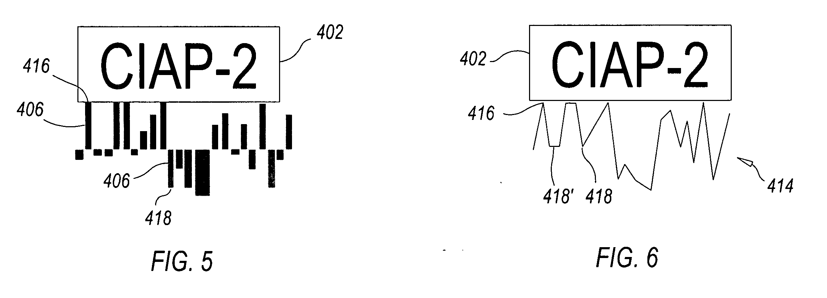

Fig. 5 is a magnified view of the node "CIAP" 402 from Fig. 4 and its associated heat strip overlay 404. Each color-encoded vertical bar 406 encodes a data value for the gene "CIAP-2" for a different experimental condition. The lengths of each bar 406, that ascends from the imaginary centerline, is proportional to the relative data value that it represents, just as the color is encoded relatively, where higher relative values for up-regulation are brighter red, as described above. Similarly, the lengths of the vertical bars that descend from the imaginary centerline, as well as their degrees of greenness, are proportional to the relative data values for down-regulation that they represent. Thus the present invention maps numerical values of the data represented into size as well as color representations. Perceptual psychology research has found size to be a better perceptual indicator of comparative quantity than color.

Fig. 6 is a magnified view of the node "CIAP" 402, similar to Fig. 5, but where the associated overlay 414 is represented in an alternative "line graph" style representation. In overlay 414, individual data values are plotted over a rectangular region underneath the nod 402, where each data value is plotted to a point 416 corresponding to the top center point of the equivalent heat strip vertical bar 406 (for up-regulated and neutral values) or to a point corresponding to the bottom center point of the equivalent heat strip vertical bar 406 for down-regulated values. Although the line graph overlay 414 in this example is not color coded, it may optionally be color coded as well, similar to the way that heatstrip 404 is color coded. For example, the lines existing above the imaginary horizontal bar representing a neutral value may be color code red, with increasing hues and intensity of the red color the further that the line extends from the neutral level. Similarly, the portions of the line that extend beneath the imaginary horizontal neutral line may be color coded green, with the intensity and or hue increasing as the line diverges further beneath the imaginary horizontal neutral line. Where the line crosses or intersects the imaginary neutral line, the color coding may be black. Also in areas where the line may run horizontally along the imaginary neutral line, these portions may also be color coded black. The flattened portion 418' signifies two peaks (conditions) with the same value, which in a heatstrip would be represented as two adjacent bars having the same depth.

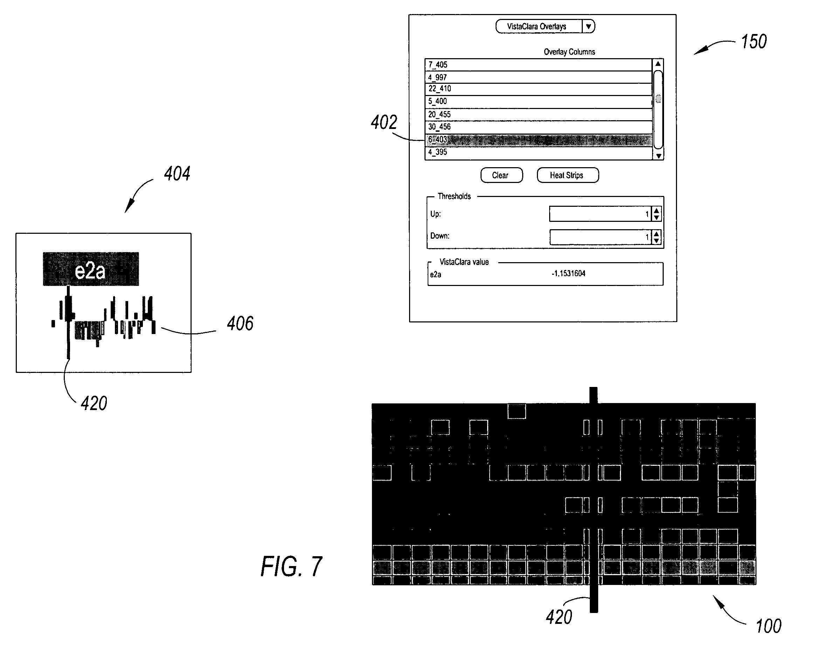

Alternative to the visualization provided in Fig. 4, nodes 402 may be color coded in the same way as described with regard to Figs. 2-3, to show a selected experimental condition, i.e., selected from one of the experimental conditions displayed in the adjacent overlay 404,414. The same experimental condition is applied for all nodes 402 relative to each node's overlay 404,414. With regard to either the visualization discussed in Fig. 4 or this alternative visualization, a cursor 420 may be provided to show the particular vertical bar 406 or peak 416 that is being displayed by color coding in the associated node 404 as shown in Fig. 7. Further optionally, visualization 400 may be linked with heat map 100 and/or a list of experimental data values 150 corresponding to the row of data values displayed in an overlay 406 or 416. By selecting or clicking on a cursor 420 in a particular overlay 406,416, this automatically displays the cursor 420 over the corresponding value in chart display 150. When a heat map 100 is linked and displayed, selection of the cursor as described, also shows the cursor 420 over the corresponding column of the experimental condition that is selected by the cursor in the overlay 406,416. Movement of the cursor 420 to another vertical bar 406 or point 416 automatically changes the color coding of node 404 to reflect the value that is newly indicated by cursor 420. Additionally, cursors in views 100 and 150 are also automatically repositioned to the corresponding positions.

Conversely, a user may wish to select a value in display 150 to automatically move the cursor of the corresponding overlay 406,416 to select the same value represented there, and, optionally, to automatically color code associated node 404 for the newly selected value. By selecting on a cursor of a particular overlay 406,416 associated with a particular node 404, the user can automatically change the display 150 to show the correct column of data that corresponds to the node currently selected. The cursor 420 in view 100 can also be changed by the user to display a different experimental condition in view 400, with the cursors on the overlays 406,416 automatically changing to reflect the change in cursor position made in view 100.

Still further, overlays 404,414 may be used as an active interface element for sorting. If the underlying data set being overlaid is sorted by experiment, such as by using some sort criteria in a separate view (see Application Serial No. 10/403,762 for detailed disclosure regarding sorting techniques), then the overlays 404,414 may be synchronized so that they reflect the same sort order of the experimental data represented. Further, a user may select one data value on an overlay 404,414, using cursor 420 and select a sort operation (form a menu bar) based on the expression value selected by cursor 420. The results of the sort are then displayed on the overlays 404,414 as well as on any additionally linked view, such as view 100, for example.

If a subset of experiments in the underlying data set are selected, such as by using a system as described in Application Serial No. 10/403,762, for example, where a view from the system, such as view 100, for example is linked with a view displaying overlays 404,414 (such as view 400, for example), then such selection also automatically filters the data that is shown in the overlays 404,414 in the linked view 400, to show only data from the selected experiments. Conversely, a ranged of experiments in an overlay 404,414 may be selected (by a technique referred to as "brushing") to select a range of experiments in the underlying dataset. Upon such selection, only the experimental data from the selected subset is displayed in each of the overlays 404,414. Also, the selection is automatically displayed on any linked views, such as view 100.

One non-limiting example of sorting and selection is as follows: a user selects a row of gene expression data from a matrix such as displayed in view 100, for example. A heat strip 404 is generated in response to the selected row, which may also be overlaid adjacent a node representative of the entity that the row of experimental data represents (such as a gene, when the data is gene expression data). The user then clicks on the generated heat strip, wherein the system displays a popup menu of functional options. From the popup menu, the user selects an option to sort the heat strip display 404 by decreasing gene expression levels. Next, the user selects the up-regulated experiments in the sorted list 150 (which is linked to heat strip 404 and thus automatically sorted by the user's selection of the sort operation. The user then selects all up-regulated experimental values in the sorted list which automatically selects the experiments in the underlying data set from which these values were taken. The heat strip 404 and all linked visualizations are then automatically updated to display only experimental data from the selected experiments and in the sort order that was resultant from the sort.

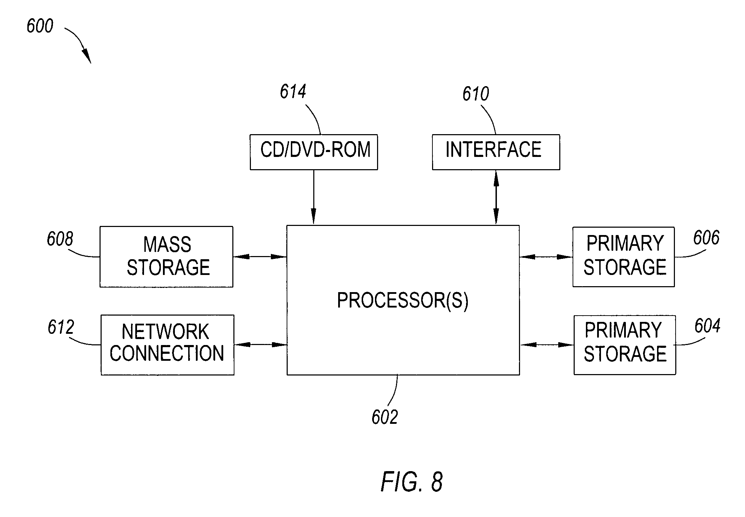

Fig. 8 illustrates a typical computer system 600 that may be used in processing events described herein. The computer system 600 includes any number of processors 602 (also referred to as central processing units, or CPUs) that are coupled to storage devices including primary storage 606 (typically a random access memory, or RAM), primary storage 604 (typically a read only memory, or ROM). As is well known in the art, primary storage 604 acts to transfer data and instructions uni-directionally to the CPU and primary storage 606 is used typically to transfer data and instructions in a bi-directional manner Both of these primary storage devices may include any suitable computer-readable media such as those described above. A mass storage device 608 is also coupled bi-directionally to CPU 602 and provides additional data storage capacity and may include any of the computer-readable media described above. Mass storage device 608 may be used to store programs, data and the like and is typically a secondary storage medium such as a hard disk that is slower than primary storage. It will be appreciated that the information retained within the mass storage device 608, may, in appropriate cases, be incorporated in standard fashion as part of primary storage 606 as virtual memory. A specific mass storage device such as a CD-ROM 614 ( or DVD-ROM, CD-RW, DVD-RW, or the like) may also pass data uni-directionally to the CPU.

CPU 602 is also coupled to an interface 610 that includes one or more input/output devices such as such as video monitors, track balls, mice, keyboards, microphones, touch-sensitive displays, transducer card readers, magnetic or paper tape readers, tablets, styluses, voice or handwriting recognizers, or other well-known input devices such as, of course, other computers. Finally, CPU 602 optionally may be coupled to a computer or telecommunications network using a network connection as shown generally at 612. With such a network connection, it is contemplated that the CPU might receive information from the network, or might output information to the network in the course of performing the above-described method steps. The above-described devices and materials will be familiar to those of skill in the computer hardware and software arts.

The hardware elements described above may implement the instructions of multiple software modules for performing the operations of this invention. For example, instructions for performing a sort of expression values may be stored on mass storage device 608 or 614 and executed on CPU 608 in conjunction with primary memory 606.

In addition, embodiments of the present invention further relate to computer readable media or computer program products that include program instructions and/or data (including data structures) for performing various computer-implemented operations. The media and program instructions may be those specially designed and constructed for the purposes of the present invention, or they may be of the kind well known and available to those having skill in the computer software arts. Examples of computer-readable media include, but are not limited to, magnetic media such as hard disks, floppy disks, and magnetic tape; optical media such as CD-ROM, CD-RW, DVD-ROM, or DVD-RW disks; magneto-optical media such as floptical disks; and hardware devices that are specially configured to store and perform program instructions, such as read-only memory devices (ROM) and random access memory (RAM). Examples of program instructions include both machine code, such as produced by a compiler, and files containing higher level code that may be executed by the computer using an interpreter.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, hardware, data, process, process step or steps, to the objective, spirit and scope of the present invention. All such modifications are intended to be within the scope of the claims appended hereto.

## Claims

1. A method of visualizing multiple data values adjacent graphical representations of entities in a diagram representing biological relationships between the entities, the method comprising the steps of:
displaying a diagram (100,200,400) of interconnected entities representing biological relationships between the entities;
providing a data set having rows of data values, each row containing values representing a single entity; and
overlaying a display (404,414) of a row of data values from the dataset on the displayed diagram (100,200,400) such that the row of data values (404,414) appears adjacent the entity (402) on the diagram that matches the entity in the data set that the row of data characterizes;
wherein the display of the row of data values is scaled so that components of the display are dimensionally proportional to numerical values of the data values taken from the data set.

2. The method of claim 1, wherein a display of a row of data values (404,414) is overlaid adjacent each entity (402) in the diagram (100,200,400) for which there is a match in the data set and for which data values are contained.

3. The method of claim 1 or 2, wherein the display of a row of data values comprises a heat strip (404).

4. The method of any of claims 1-3, where in the display (404,414) of the row of data values is color coded proportionally to the numerical values of the data values taken from the data set.

5. The method of any of claims 1-4, wherein the display (404,414) of the row of data values is scaled in at least one dimension proportionally to the numerical values of the data values in the row taken from the data set.

6. The method of any of claims 1,2, 4 or 5, wherein the display of a row of data values comprises a line graph visualization (414).

7. The method of any of claims 1-6, further comprising selecting a data value from the row of data values and color coding a graphical representation of the adjacent entity (402) to represent the numerical value of the selected data value.

8. The method of claim any of claims 1-7, further comprising linking the overlaid display (400) with at least one of a visualization of the data set (100) and a visualization of data values of the selected row of data (150); wherein an operation performed on the overlaid display is automatically performed on the at least one linked visualization.

9. The method of claim 8, wherein an operation performed on one of the linked visualizations (100,150) is automatically performed on the overlaid display (400) and any other linked visualization (100,150).

10. The method of any of claims 1-9, further comprising sorting data values in the overlaid display, based upon user selection of a data value in the overlaid display.

11. The method of any of claims 1-10, further comprising selecting a subset of the values in the overlaid display, and displaying only rows of data from the data set of which the selected values are members.

12. The method of any of claims 8-11, further comprising user selection of a data value from the row of data values using a cursor, wherein the data value is automatically identified in the linked visualization of data values of the selected row of data by another cursor in the linked visualization.

13. The method of any of claims 8-12, further comprising performing a sort of the data in one of the linked visualizations; and
automatically displaying data in the overlaid display of the row of data values in an order resultant from the sort.

14. The method of any of claims 3-13, further comprising selecting a subset of columns of data from the data set in a visualization of the data set, and automatically displaying only data values in the overlaid display of the row of data values that are also members of the selected subset of columns.

15. A method comprising forwarding a result obtained from the method of any of claims 1-14 to a remote location.

16. A method comprising transmitting data representing a result obtained from the method of any of claims 1-14 to a remote location.

17. A method comprising receiving a result obtained from a method of any of claims 1-14 from a remote location.

18. A visualization graphic (404,414) for representing a row of data values from a dataset on a displayed diagram such that the row of data values appears adjacent an entity (402) on the diagram that matches the entity in the data set that the row of data characterizes, said visualization graphic comprising a graphical representation of each data value in the row of data values represented, wherein each graphical representation is scaled dimensionally proportional to a numerical value of the data value that it represents, as taken from the data set.

19. The visualization graphic of claim 18, wherein the visualization graphic comprises a heat strip (404).

20. The visualization graphic (404,414) of claim 18 or 19. wherein the graphical representations are color coded proportionally to the numerical values of the data values taken from the data set.

21. The visualization graphic of claim 18 or 20, wherein the visualization graphic comprises a line graph visualization (414).

22. A system for visualizing multiple data values adjacent graphical representations of entities in a diagram (100,200,400) representing biological relationships between the entities (402), comprising:
means for displaying a diagram (100,200,400) of interconnected entities representing biological relationships between the entities;
means for providing a data set having rows of data values, each row containing values representing a single entity; and
means for overlaying a display (404,414) of a row of data values from the dataset on the displayed diagram (100,200,400) such that the row of data values appears adjacent the entity (402) on the diagram that matches the entity in the data set that the row of data characterizes;
wherein the display (404,414) of the row of data values is scaled so that components of the display are dimensionally proportional to numerical values of the data values taken from the data set.

23. A computer readable medium carrying one or more sequences of instructions from a user of a computer system for visualizing multiple data values adjacent graphical representations of entities in a diagram representing biological relationships between the entities, wherein the execution of the one or more sequences of instructions by one or more processors cause the one or more processors to perform the steps of:
displaying a diagram (100,200,400) of interconnected entities representing biological relationships between the entities;
accessing a data set having rows of data values, each row containing values representing a single entity; and
overlaying a display (404,414) of a row of data values from the dataset on the displayed diagram (100,200,400) such that the row of data values appears adjacent the entity (402) on the diagram that matches the entity in the data set that the row of data characterizes;
wherein the display (404,414) of the row of data values is scaled so that components of the display are dimensionally proportional to numerical values of the data values taken from the data set.
